# EUROPÄISCHE PATENTSCHRIFT

(11) **EP 1 365 016 B1**
(45) Veröffentlichungstag und Bekanntmachung des Hinweises auf die Patenterteilung: **11.07.2007**
(21) Anmeldenummer: 03011374.0
(22) Anmeldetag: 19.05.2003
(51) Int. Cl.: C12M 3/00, C12N 15/87

(54) **Verfahren und Einrichtung zum Transferieren medizinisch wirksamer Stoffe in Zellen**
Process and apparatus to transfer medical actif compounds into cells
Procédé et appareil pour le transfert de composés médicaux actifs dand des cellules

(30) Priorität: 24.05.2002 DE 10223196
(43) Veröffentlichungstag der Anmeldung: 26.11.2003
(73) Patentinhaber: Dornier MedTech Systems GmbH, 82234 Wessling (DE)
(72) Erfinder: Ueberle, Friedrich, Dr., 82205 Gilching (DE)
(74) Vertreter: Grünecker, Kinkeldey, Stockmair & Schwanhäusser Anwaltssozietät

(56) Entgegenhaltungen:
- WO-A-01/48181
- WO-A-89/02464
- DE-A- 19 820 466
- DE-A- 19 834 612
- US-A- 5 204 253

## Beschreibung

Verfahren und Einrichtung zum Transferieren medizinisch wirksamer Stoffe in Zellen.

Die Erfindung bezieht sich auf ein Verfahren und auf eine Einrichtung zum Transferieren medizinisch wirksamer Stoffe (Agenzien) in Form von Molekülen - wie zum Beispiel DNA, - in Zellen von Menschen, Tieren und/oder Pflanzen mittels durch Schallenergie bewirkte Kavitation, die durch entsprechende Intensität der Schallenergie erzeugt wird, und zwar in vitro wie auch ex vivo und in vivo.

Eine einschlägige Vorrichtung zum Transfer von Molekülen in Zellen von menschlichen, tierischen und/oder pflanzlichen Lebewesen ist in der PCT-Anmeldung mit der Internationalen Veröffentlichungsnummer WO 01/48181 A2 beschrieben und dargestellt. Die hierdurch bekanntgewordene Vorrichtung, die gegenüber einem umfangreichen einschlägig bekannten Stand der Technik, der ebenfalls in der genannten Vorveröffentlichung zitiert und diskutiert wurde, bereits abgegrenzt ist, bezieht sich auf den Transfer von Molekülen in Zellen, wobei ein Medium, in dem sich die zu transferierenden Moleküle und die Zielzellen befinden, im Fokusbereich einer Quelle akustischer Impulse diesen akustischen Impulsen aussetzbar ist.

Das besondere dieser Vorrichtung besteht darin, dass
- die Quelle einen linienförmigen Fokusbereich aufweist, in welchem die akustischen Pulse einen vorgegebenen Druck oder Unterdruck und/oder eine vorgegebene Intensität überschreiten und
- eine hohlzylindrische Vorrichtung vorgesehen ist, in die radial von außen eine Vielzahl von gleichphasigen Schallimpulsen eingeleitet wird, wobei im Bereich um die Rotationsachse des Hohlzylinders eine reproduzierbare Zone transienter Kavitationsereignisse erzeugt wird, und dass schließlich
- eine weitere Vorrichtung vorgesehen ist, durch welche eine Relativbewegung zwischen dem Medium und dem Fokusbereich ausführbar ist.

Die zu behandelnden Zielzellen werden durch Kavitation transient permeabel gemacht für die zu transferierenden Moleküle der medizinischen Wirkstoffe. Dabei ist darauf zu achten, daß die Kavitation möglichst effizient gesteuert wird, d.h. die Zielzellen sollen durch die Kavitation gerade hinreichend transient permeabel gemacht werden, das heißt sie sollen keinesfalls durch Übersteuerung der Kavitation beschädigt werden, vor allem nicht auf Dauer geschädigt oder gar zerstört werden.

Der Erfindung liegt daher die Aufgabe zugrunde, ein Verfahren und eine Einrichtung zur Steuerung der Kavitation vorzuschlagen, wodurch die vorgenannte Zielsetzung in möglichst einfacher und zuverlässiger Weise realisiert werden kann.

Gelöst ist diese Aufgabe im wesentlichen dadurch, dass mittels geeigneter Sensoren - vor allem mittels Schallsensoren - die Lebensdauer der Kavitationsblasen bestimmt wird, die dann ihrerseits als Kriterium bzw. als Funktion zur Steuerung der Schallintensität und damit auch der Kavitationsintensität fungiert.

Der Erfindung liegt die beachtliche Erkenntnis zugrunde, dass hinsichtlich der Kavitationsblasen die Zeit zwischen deren Entstehung und ihrem Kollaps ein direkter Indikator ist für die in der Kavitationsblase umgesetzte Energie. Dies bedeutet, dass mit der Ermittlung bzw. der Vorgabe der Lebensdauer der Kavitationsblasen auch die für die jeweilige Transfektion von Zielzellen optimal einzusetzende Energie zuverlässig steuerbar ist.

Zur praktischen Lösung der erfindungsgemäßen Aufgabe wird vorgeschlagen, den Schallwandler mit Sensoren zu kombinieren bzw. zu bestücken, die in der Lage sind, die vor allem beim Zerfall der Kavitationsblasen entstehenden Schallsignale zu registrieren, sie an eine Einrichtung weiterzuleiten, die sie in elektrische Signale umwandelt, die dann ihrerseits direkte Rückschlüsse auf die Kavitationsaktivität erlauben.

Aus der Auswertung der aufgenommenen Schallsignale lassen sich neben der Kavitationsaktivität auch die Lebensdauer der Kavitationsblasen, der Ort ihrer Entstehung, die Position von Probenröhrchen etc. bestimmen.

Des weiteren können die im Rahmen der Erfindung aufgenommenen Signale auch zur Darstellung von Bildern ausgewertet werden, aus denen der genaue Ort von Kavitationen und Schallkonzentrationen z.B. auf einem eingebrachten Pflanzenblatt erkennbar ist, womit dann auch die Position der transfizierten Zellen bekannt ist.

Auch eine Regelung der Ansteuerungsfunktion und/oder der Stärke, der Pulsfolge ist möglich, um die Wirkung beim Transferieren von Zellen zu optimieren.

Die Auslösung der Schallsignale erfolgt über eine Steuereinrichtung, die z.B. auch bestimmte Serien, Pulszahlen etc. programmiert oder als Vorwahl abgeben kann.

Durch einen zusätzlich vorgesehenen Thermosensor kann das erfindungsgemäße Verfahren sinnvoll weiter optimiert werden. Der Thermosensor erlaubt die exakte Bestimmung der Probentemperatur, so dass die umgebende Flüssigkeit temperiert werden kann oder auch die Pulsparameter (Folgefrequenz, Burstlänge, Amplitude) so eingestellt werden können, dass die empfindlichen Zellen nicht überhitzt werden.

Auf diese Weise ist es auch möglich, das zu beschallende Gut zunächst in Unterkühlung zu halten - zB. mit kaltem Wasser o.ä., ggf. sogar mit flüssigem Stickstoff- und nur die gewünschte Zielstelle durch die Schallsignale gezielt zu erwärmen. Es kann auch eine geeignete Signalfolge erzeugt werden, die durch Überlagerung zunächst die Erwärmung verursacht und im richtigen Moment die transfizierenden Schallsignale auslöst.

Bei einer Flüssigkeit mit bekannten Eigenschaften, im besonderen hinsichtlich des Sauerstoffgehaltes, der Viskosität und der Reinheit können typische Kollapszeiten der Kavitationsblasen in Abhängigkeit von der Intensität der Schallwandler empirisch ermittelt werden. Diese Messwerte können dann als Ausgangswerte für praktische Anwendungsfälle verwendet werden.
Im umgekehrten Falle ist es auch möglich, anhand ermittelter Kollapszeiten Aussagen über den Zustand bzw. die Beschaffenheit bestimmter Flüssigkeiten zu treffen.
Des weiteren ist es bekannt, dass Kavitation in Flüssigkeiten durch Überdruck be- bzw. verhindert werden kann. Auch diese Erkenntnis kann zusätzlich zur Steuerung der Kavitation herangezogen werden.

In den Abbildungen sind das erfindungsgemäße Verfahren wie auch Einrichtungen zur Durchführung des Verfahrens anhand von Ausführungsbeispielen zeichnerisch erläutert.

Es zeigen:
- Abb. 1: in graphischer Darstellung den grundsätzlichen Radius-Verlauf einer Kavitationsblase über der Zeit und zwar von ihrer Entstehung bis zu ihrem Zerfall, mit dem jeweils immer kleinere Kavitationsblasen im Gefolge entstehen.
- Abb. 2: eine Einrichtung zur Durchführung des erfindungsgemäßen Verfahrens mit einem zylinderförmigen Schallwandler.
- Abb. 3: die Einrichtung gemäß Abb. 2 in einem anderen Beschallungszustand.
- Abb. 4: eine Einrichtung zur Durchführung des erfindungsgemäßen Verfahrens mit einem Schallwandler in schalenförmiger Ausgestaltung .
- Abb. 5: die Einrichtung gemäß Abb. 4 in einem anderen Beschallungszustand.
- Abb. 6: im Teil A die Entwicklung bzw. den Verlauf der relativen Radien der Kavitationsblasen über der Zeit; im Teil B ist anhand eines Druck-/ Zeitdiagramms erläutert, wie zeitgleich mit den kollabierenden Kavitationsblasen gemäß Teil A entsprechend neue Stoßwellen freigesetzt werden bzw. entstehen; der Teil C zeigt schließlich schematisch auf, zu welchen Zeiten die Kollapssignale am Ort der Sensoren empfangen werden können.
- Abb. 7: die Kollapszeit als Funktion der Schallwandler-Steuerspannung in zwei verschiedenen Medien.
- Abb. 8: eine Schaltung zur Ansteuerung eines Schallwandlers.

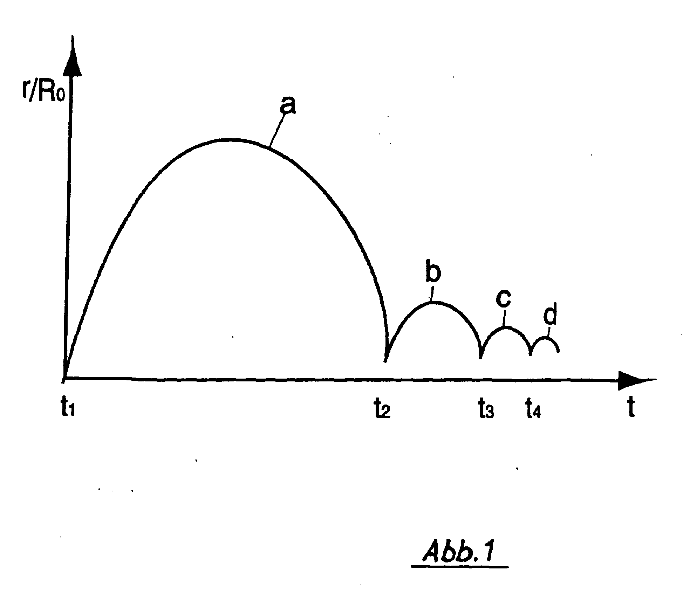

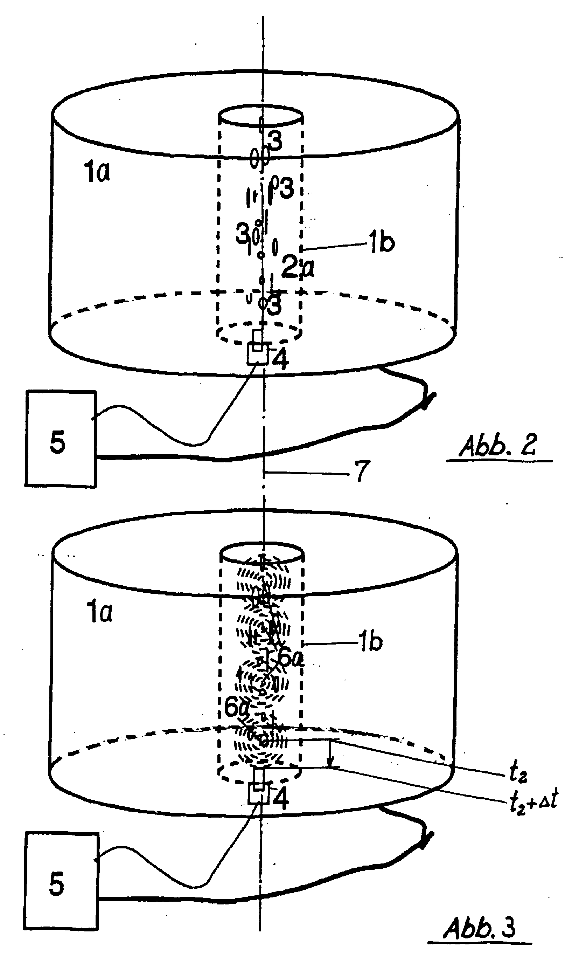

Gemäß Abb. 1 entsteht eine Kavitationsblase zum Zeitpunkt t₁. Bis zu ihrem Kollaps im Zeitpunkt t₂ entspricht ihr Radius dem Kurvenverlauf a über der Zeit t, wobei hier konkret der relative Kavitationsblasenradius r, bezogen auf den Radius Ro des Blasenkeims als Funktion der Zeit t dargestellt ist.
Das Erzeugen einer Kavitationsblase zum Zeitpunkt t₁ geschieht durch das Auftreffen der Schallwelle auf einen Blasenkeim im Medium 2a (sh. Fig.2) oder durch das Überschreiten der Kavitationsschwelle bei entsprechender Schallintensität. Die bei t₁ entstandene Kavitationsblase kollabiert wieder im Zeitpunkt t₂. Immer kleinere Kavitationsblasen mit den Kurvenverläufen b,c,d, etc. entstehen im Gefolge der jeweils vorher kollabierten Blase und zwar durch deren jeweils beim Kollabieren freigesetzte Energie.

Grundsätzlich kann festgestellt werden, dass mit zunehmendem Radius einer Kavitationsblase auch ihre Lebensdauer zunimmt. Anders ausgedrückt bedeutet dies, dass Kavitationsblasen mit größeren Radien und mit entsprechend größerem Energiegehalt dem entsprechend später kollabieren. Demnach ist die Kollapszeit ein analoger Indikator für die jeweils in der betreffenden Kavitationsblase umgesetzte Energie.

In Abb. 2 ist ein Schallwandler 1a gezeigt, der in seinem Inneren in homogener, zylinderförmiger Anordnung beispielsweise eine Vielzahl von nicht gesondert dargestellten Piezoelementen besitzt, deren Kraftlinien sich im Bereich der zentralen Mittellinie 7 des Schallwandlers 1a treffen. Damit bildet die Mittellinie 7 auch gleichzeitig die Zentrallinie eines Linien-Fokusbereiches im Innern des zylinderförmigen Hohlraums 1b des Schallwandlers 1a. Bei Beschallung durch diesen können somit entlang des Fokusbereiches im Medium 2a Kavitationsblasen erzeugt werden. Dabei kann sich das Medium 2a direkt in dem Hohlraum 1b oder in einem in diesen einsetzbaren Probenröhrchen befinden. In das Medium 2a sind zu behandelnde Zellen und/oder Moleküle 3 eingebracht, die im Rahmen der Erfindung einer äußerst genau dosierbaren Beschallung ausgesetzt werden können. Die Kollapszeiten der dabei entstehenden Kavitationsblasen werden von einem Sensor 4 registriert, der seine Signale an eine elektronische Einheit 5 zur Steuerung des Schallwandlers 1a weiterleitet.

Die Einrichtung gemäß Abb. 2 befindet sich im nicht beschallten Zustand. Im Moment des Kollaps einer Kavitationsblase wird entsprechend der Abb. 3 ein Schallsignal 6a ausgesandt, das - wie ebenfalls aus der Abb. 3 ersichtlich - nach der Zeit t₂ + Δt einen Sensor 4 erreicht, der es zur Weiterverarbeitung an die elektronische Einheit 5 weiterleitet.

Aufgrund von Laborversuchen kann festgestellt werden, dass der Linien-Fokusbereich mit der Mittellinie 7 einen Durchmesser von ca. 2 mm hat. Demzufolge sollten die Behältnisse, beispielsweise ein Probenröhrchen aus akustisch gut leitendem Kunststoff, z.B. aus Polyurethan, in welchem sich das Medium 2a mit den zu transferierenden Zielzellen und/oder Molekülen 3 befindet, einen maximalen Durchmesser von nur 3-4 mm haben, damit bei einer Beschallung möglichst viele Zielobjekte erfasst werden. Gefördert wird letzteres auch dadurch, dass während der Beschallung durch diese im Medium 2a eine gewisse Durchwirbelung entsteht.

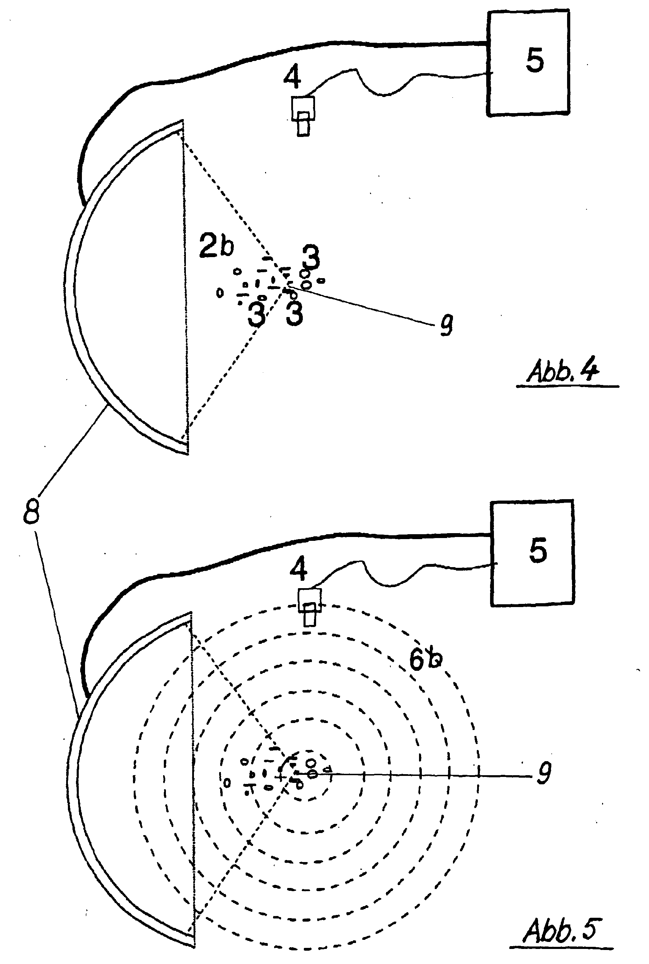

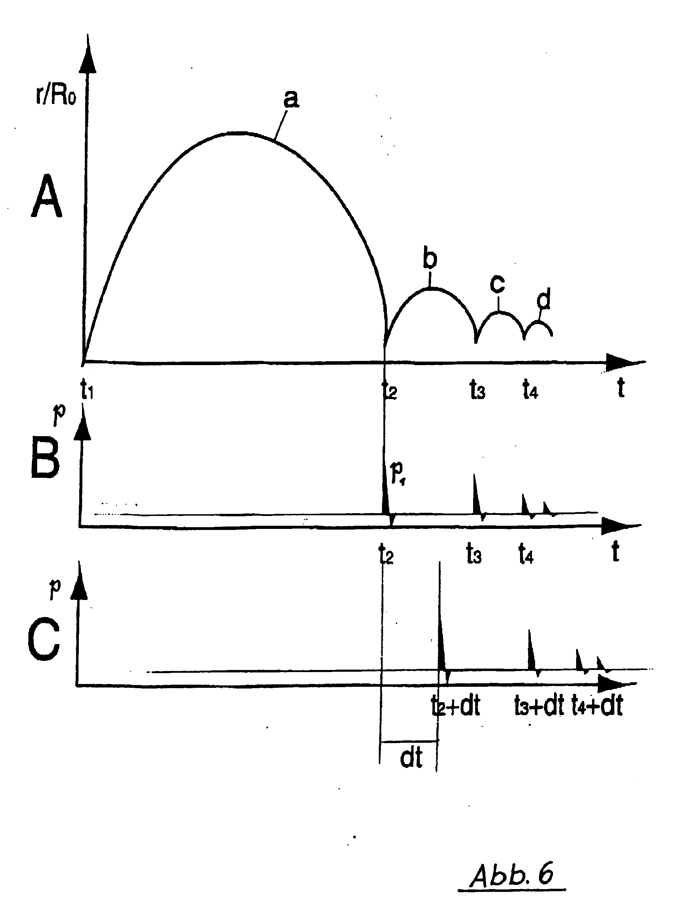

Die Einrichtung gemäß der Abb. 4 besitzt einen schalenförmigen Schallwandler 8, dessen Kraftlinien im Bereich 9 punktförmig fokussieren. Wenn das Medium 2b gemäß Abb. 5 einer Beschallung unterzogen wird, bei der Kavitationsblasen entstehen, dann erreichen auch hier die von den kollabierenden Kavitationsblasen ausgesendeten Schallsignale 6b Schallsignale 6b nach einer Zeit t₂ + Δt den Sensor 4. Der Schallwandler 8 kann in homogener Anordnung mit Piezoelementen bestückt sein oder auch in Form einer sogenannten Kalotten-EMSE realisiert sein. Denkbar wäre hier auch ein magnetostriktiver Schallwandler.

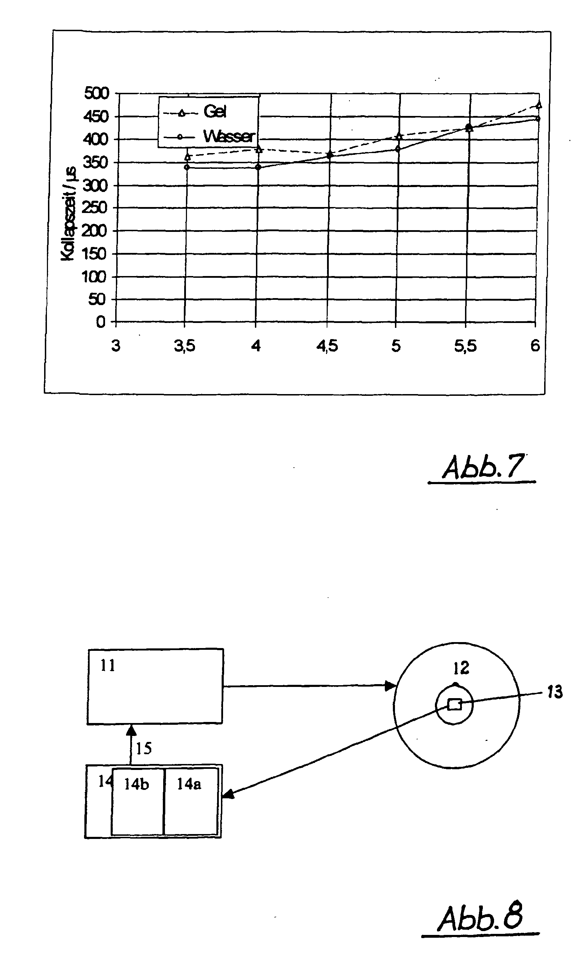

In Abb. 6 zeigt der Teil A den relativen Kavitationsblasenradius r, bezogen auf den Radius des Blasenkeims R₀, als Funktion der Zeit t. Es kann davon ausgegangen werden, dass die Blasenradien R₀ bereits nach wenigen Schallpulsen sehr ähnlich sind und somit eine recht einheitliche Kollapszeit aller Blasen erreicht wird.

Zum Zeitpunkt t₁ wird die Blase von einem akustischen Impuls, zum Beispiel von einer Stoßwelle angeregt und vergrößert dadurch ihren Radius. Dabei diffundiert in der Flüssigkeit gelöstes Gas und Dampf der Flüssigkeit in ihr Inneres. Nach einer Zeit, die sowohl von den Umgebungsbedingungen - zum Beispiel vom statischen Druck und der Viskosität - wie von dem anregenden Impuls abhängt, verringert sich der Blasenradius mit zunehmender Geschwindigkeit und die Blase kollabiert zum Zeitpunkt t₂. Im Augenblick des Kollaps t₂ wird die aufgespeicherte Energie zu einem Teil in Form einer Stoßwelle freigesetzt. Dies ist im Teil B der Abb. 6 am Ende der Blase a zum Zeitpunkt t₂ als Druckstoß p₁ zeichnerisch dargestellt. Die hierdurch erzeugte Stoßwelle breitet sich als sphärische Schallwelle im Medium aus und erreicht zum Zeitpunkt t₂ + Δt den Meß-Sensor. Dieser ist in einer Entfernung x vom Ort des erwarteten Blasenkollaps angebracht. Die Kollapssignale erreichen ihn daher nach einer Zeit Δt = x/c, wobei c die Schallgeschwindigkeit im Medium ist. Die Kollapssignale am Ort des Sensors sind im Teil C der Abb. 6 dargestellt. Je nach Beschaffenheit des Mediums kann sich die Abfolge von Radius-Vergrößerung und Kollaps mehrmals wiederholen, wobei - vergleichbar mit einem springenden Ball - durch Dämpfungseffekte und Abstrahlverluste für die betreffende Blase immer weniger Energie verfügbar ist und damit der Radius und die Zeit bis zum jeweils nächsten Kollaps immer geringer wird.
Bei einer Einrichtung mit fokussiertem Wandler gemäß den Abbildungen 4 und 5 sind in der Regel die Orte von Kavitationen auf den Fokusbereich der Schallquelle beschränkt und damit etwa gleich weit vom Sensor entfernt. Dadurch erhält man ein recht kurzes Schallsignal.
Im Falle des linienförmigen Fokus gemäß den Abbildungen 2 und 3 werden bei Anordnung des Sensors in Achsnähe die Kollapssignale der Blasen zu unterschiedlichen Zeiten am Sensor eintreffen. In diesem Fall empfängt man ein länger andauerndes, rauschähnliches Kollapssignal. Es genügt hier, den Zeitpunkt des Einsetzens dieses Signals erfindungsgemäß auszuwerten, da zu erwarten ist, dass es von einer dem Sensor nächstliegenden Blase stammt, und dass die allermeisten übrigen Blasen in etwa gleichzeitig entstehen und wieder zerfallen.

Die folgende Beschreibung bezieht sich auf eine Anordnung mit fokussiertem Schallwandler (Fokusentfernung 200 mm) gemäß Abbildung 4.
Dabei wird derselbe Schallwandler, der zur Erzeugung der zur Kavitation führenden Schallimpulse dient, auch zum Empfang der Kavitationssignale verwendet.

Mit Hilfe eines Hochspannungstastkopfes ist es möglich, den Spannungsverlauf am Piezowandler zu verfolgen. Da die Piezoelemente auch empfangene akustische Signale wieder in Spannungssignale verwandeln, kann man zum Beispiel Echos von Gegenständen im Schallfeld empfangen. Da der Wandler sehr stark fokussiert, werden hauptsächlich Echos aus der Fokusregion mit großer Amplitude wiedergegeben. Ebenso ist es möglich, die Druckwellen zu empfangen, die von zerfallenden Kavitationsblasen insbesondere im Fokusbereich abgestrahlt werden.

Dabei wurde insbesondere das Signal ab 256 µs nach der Auslösung der Schallpulse analysiert. Diese 256 us entsprechen bei einem Wandlerradius von 195 mm und einer Schallgeschwindigkeit von 1523 m/s (bei 35°C Wassertemperatur) exakt der Laufstrecke von der Wandleroberfläche zum Fokus und wieder zurück. 128 µs nach seiner Auslösung gelangt das Wandler-Schallsignal zum Fokus. Wird das Signal dort an einem Hindernis reflektiert, so kommt es dementsprechend nach weiteren 128 µs wieder am Wandler an.

Die Zeitabfolge in Wasser ist - gemessen - wie folgt:

| **Zeit** | **Signalort** | **Laufstrecke** |
|---|---|---|
| 0 | Wandleroberfläche | 0 |
| 128 µs | Fokus | 195 mm |
| 258 µs | Wandleroberfläche (1. Reflex) | 390 mm |
| 388 +- 2 µs | Fokus | 585 mm |
| 517 +- 2 µs | Wandleroberfläche (2. Reflex) | 780 mm |
| 644 +- 2 µs | Fokus | 975 mm |
| ................... | | |

Bringt man ein Gelmaterial in den Schallweg (Dicke 80 mm, Schallgeschwindigkeit 1465 m/s, Dämpfung 0,53 dB/cmMHz) zwischen Wandler und Fokus, so verlängern sich die Laufzeiten auf 132 µs, 264 µs, 392 µs, 528 µs und 658 µs.
Eine Kavitationsblase entsteht, wenn der Unterdruck die Zerreißfestigkeit des Wassers überschreitet, was insbesondere im Fokus der Fall ist. Dort entsteht eine Blase, die je nach ihrer Größe nach einer bestimmten Zeit wieder kollabiert. Dabei wird eine Stoßwelle generiert, die Blasengröße und damit die Kollapszeit ist intensitätsabhängig.
Vom Schallwandler wird dann der Blasenkollaps ebenfalls empfangen, und zwar 128 µs später entsprechend dem Laufweg vom Entstehungsort zum Wandler.

In einem praktischen Beispiel entsteht in Wasser eine Kavitation, was durch Messung der Wandlersignale nachgewiesen werden kann. In reinem Wasser wird zunächst die Reflexion der Primärwelle an den gerade entstehenden Blasen nach 262 µs gefunden. Die Amplituden der Signale schwanken dabei stark, da die Blasenbildung ein nichtlineares, statistisches Phänomen ist. Danach kommt nach 340 µs zunächst recht schwach das Echo der 255 mm vom Wandlerzenit entfernten Wasseroberfläche, dann das Kollaps-Signal (im Beispiel nach 444 µs) und nach 530 µs ein Signal, das als Wiederholungsecho zwischen Wandleroberfläche und der noch bestehenden Kavitationsblasen im Fokus gedeutet werden kann; der von der Blase primär generierte Schall war ja nach Reflexion am Wandler nach 393 µs wieder im Fokus, wo die Blase noch lebte (Kollaps-Signal bei 444 µs kommt 178 µs nach Entstehungssignal).

Die Tatsache, daß das erste Signal nach 262 µs ankommt, weist daraufhin, daß die Kavitationsentstehung genau im geometrischen Fokus der Wandlerstruktur bei 200 mm erfolgte.

Mit dem Gelkissen im Schallweg werden kaum Echos von der Wasseroberfläche sichtbar, und auch die Mehrfachechos sind stark gedämpft. Das Blasenkollaps-Signal ist dennoch deutlich zu detektieren. Gegenüber Wasser wirkt sich wiederum die geringere Schallgeschwindigkeit im Gel verzögernd aus.

Die Abbildung 7 zeigt die Kollapszeit als Funktion der Wandler-Steuerspannung, gemessen in zwei verschiedenen Medien nach der oben angegebenen Methode.

Die Abbildung 8 zeigt eine Blockschaltung mit einer Schaltung 11 zur Ansteuerung des Schallwandlers, beispielsweise in Form einer Impulsschaltung. Ein Schallwandler 12 weist in seinem Inneren einen Schallsensor 13 für die Kollapssignale auf. Eine Auswerte-Schaltung 14 besteht im wesentlichen aus einem Vorverstärker und Zeittor 14a sowie aus einem Schwellwertschalter 14b. Eine Triggerleitung 15 dient zur Auslösung des Steuersignals für den Schallwandler.

## Patentansprüche

1. Verfahren zum Transferieren medizinisch wirksamer Stoffe in Form von Molekülen in lebende Zellen von Menschen, Tieren und/oder Pflanzen mittels durch Schallenergie bewirkte Kavitation, und zwar in vitro wie auch ex vivo und in vivo, **dadurch gekennzeichnet, dass** über geeignete Sensoren die Lebensdauer der Kavitationsblasen bestimmt wird, die dann ihrerseits als Kriterium zur Steuerung der Schallintensität und damit der Kavitationsintensität fungiert.

2. Einrichtung zur Durchführung des Verfahrens nach Anspruch 1, bestehend aus einem Schallwandler, aus einem Medium zur Schallübertragung, in welchem sich die zu transferierenden Moleküle und die Zielzellen befinden und zwar im Fokusbereich des Schallwandlers, wo die Zielzellen gezielten, die Kavitation erzeugenden akustischen Pulsen aussetzbar sind, **dadurch gekennzeichnet, dass** der Schallwandler (1a; 8) über Sensoren (4) mit einer Elektronik (5) zur Erkennung der Kollapszeiten der Kavitationsblasen und zur Steuerung des Schallwandlers (1a; 8) in Abhängigkeit von den Kollapszeiten verbunden ist.

3. Einrichtung nach Anspruch 2, **dadurch gekennzeichnet, dass** der Schallwandler (1a; 8) direkt mit Schallsensoren bestückt ist.

4. Einrichtung nach Anspruch 2, **dadurch gekennzeichnet, dass** der Schallwandler (1a; 8) so ausgebildet ist, dass er durch manuelles und/oder automatisches Umschalten als Sensor fungieren kann.

5. Einrichtung nach einem oder mehreren Ansprüchen 2 bis 4, **dadurch gekennzeichnet, dass** der zentrale Hohlraum (1b) des Schallwandlers (1a) bzw. ein darin befindliches Probenröhrchen einen maximalen Durchmesser von 3 bis 4 mm hat

6. Einrichtung nach einem oder mehreren der Ansprüche 2 bis 5, **dadurch gekennzeichnet, dass** das Medium (2a, 2b), in welchem sich die zu behandelnden Zellen und Moleküle befinden, durch Thermosensoren überwacht ist, um einerseits eine Überhitzung zu vermeiden, und um zum anderen die Beschallung bei der richtigen bzw. bei der angemessenen Temperatur vorzunehmen.

## Claims

1. A method for transferring medically effective substances in the form of molecules into living cells of humans, animals and/or plants by means of cavitation effected by acoustic energy, namely in vitro, ex vivo and/or in vivo, **characterized in that** the lifetime of cavitation bubbles is determined by using suitable sensors, which then, for its part, acts as a criterion for controlling the acoustic intensity and thus the cavitation intensity.

2. An apparatus for performing the method according to Claim 1, consisting of an acoustic transformer; a medium for sound transmission in which the molecules to be transferred and the target cells are located, namely in the focusing area of the acoustic transformer where the target cells can be exposed to specific acoustic pulses generating the cavitation, **characterized in that** the acoustic transformer (1a; 8) is connected via sensors (4) to an electronic system (5) for detecting the collapse times of cavitation bubbles and for controlling the acoustic transformer (1a; 8) as a function of the collapse times.

3. The apparatus according to Claim 2, **characterized in that** the acoustic transformer (1a; 8) is fitted directly with acoustic sensors.

4. The apparatus according to Claim 2, **characterized in that** the acoustic transformer (1a; 8) is configured such that it can act as a sensor by manual and/or automatic switching over.

5. The apparatus according to one or a plurality of Claims 2 to 4, **characterized in that** the central hollow space (1b) of the acoustic transformer (1a) or a test tube contained therein has a maximum diameter of 3 to 4 mm.

6. The apparatus according to one or a plurality of Claims 2 to 5, **characterized in that** the medium (2a, 2b) in which the cells to be treated and molecules are located is monitored by thermosensors in order to prevent overheating on the one hand and to perform application of sound at the correct or appropriate temperature on the other hand.

## Revendications

1. Procédé de transfert de composés médicaux actifs sous la forme de molécules dans des cellules humaines, animales et/ou végétales vivantes, par cavitation provoquée par énergie acoustique, à savoir in vitro, tout comme ex vivo et in vivo, **caractérisé en ce que**, la durée de vie des bulles de cavitation, qui fait fonction par la suite de critère pour la commande de l'intensité acoustique et donc de l'intensité de cavitation est déterminée par des capteurs adaptés.

2. Dispositif pour la réalisation du procédé selon la revendication 1, composé d'un transducteur acoustique, d'un milieu de transmission acoustique, dans lequel se trouvent les molécules à transférer et les cellules cibles, à savoir dans la zone de focalisation du transducteur acoustique, dans lequel les cellules cibles peuvent être exposées à des impulsions acoustiques adéquates, générant la cavitation, **caractérisé en ce que** le transducteur acoustique (1a ; 8) est relié par l'intermédiaire de capteurs (4) avec une électronique (5) pour la reconnaissance des temps d'affaissement des bulles de cavitation et pour la commande du transducteur acoustique (1a ; 8) en fonction des temps d'affaissement.

3. Dispositif selon la revendication 2, **caractérisé en ce que** le transducteur acoustique (1a ; 8) est directement équipé de capteurs acoustiques.

4. Dispositif selon la revendication 2, **caractérisé en ce que** le transducteur acoustique (1a ; 8) est conçu de sorte à pouvoir faire fonction de capteur, par commutation manuelle et/ou automatique.

5. Dispositif selon l'une quelconque ou plusieurs des revendications 2 à 4, **caractérisé en ce que** la cavité centrale (1b) du transducteur acoustique (1a) ou d'un tube à essai situé dans ce dernier a un diamètre maximum de 3 à 4 mm.

6. Dispositif selon l'une quelconque ou plusieurs des revendications 2 à 5, **caractérisé en ce que** le milieu (2a, 2b) dans lequel se trouvent les cellules et molécules à traiter est surveillé par des thermocapteurs, pour éviter d'une part une surchauffe et pour effectuer d'autre part l'irradiation acoustique à la bonne température ou température adéquate.
